# EUROPEAN PATENT APPLICATION

(11) **EP 4 831 912 A1**
(43) Date of publication of application: **28.10.2026**
(21) Application number: 25398013.0
(22) Date of filing: 24.04.2025
(51) Int. Cl.: G06T 7/00, G06T 7/50

(54) **GENERATING THREE-DIMENSIONAL (3D) REPRESENTATIONS OF A PATIENT'S ANATOMY FROM RADIOGRAPHIC IMAGES**

(71) Applicant: PEEK HEALTH, S.A., 4719-005 Braga (PT)
(72) Inventor: Ribeiro, João Pedro de Araújo, 4705-460 Celeirós, Braga (PT); Silva, Sara Isabel Azevedo da, 4705-460 Celeirós, Braga (PT); Campos, Jaime Frederico Bastos de, 4715-586 Braga (PT)
(74) Representative: Fish & Richardson P.C.

(57) **Abstract**

Generating three-dimensional (3D) representations of a patient's anatomy from radiographic images. A computer-implemented method can include receiving a radiograph that represents a portion of a joint, inferring a 3D structure of the represented portion of the joint from the radiograph, registering the inferred 3D structure of the represented portion of the joint with a weight-bearing radiograph of the portion of the joint; and outputting information characterizing the registration of the inferred 3D structure with the weight-bearing radiograph.

## Description

### TECHNICAL FIELD

This invention relates to generating three-dimensional (3D) representations of a patient's anatomy from radiographic images. In general, the three-dimensional (3D) representations are generated using machine-learning systems.

### BACKGROUND

Orthopedic surgery is intended to address conditions in the musculoskeletal system. Orthopedic surgery is intended to improve joint function, relieve pain, and/or delay or prevent damage to the joint. For example, osteotomy refers generally to orthopedic surgeries that involve cutting, reshaping, and/or grafting bone, e.g., as part of a process for shortening, lengthening, or aligning the bone. When osteotomies are performed to change the alignment of a bone, the changed alignment is relative to a joint, e.g., to remove deformities or misalignments at the joint.

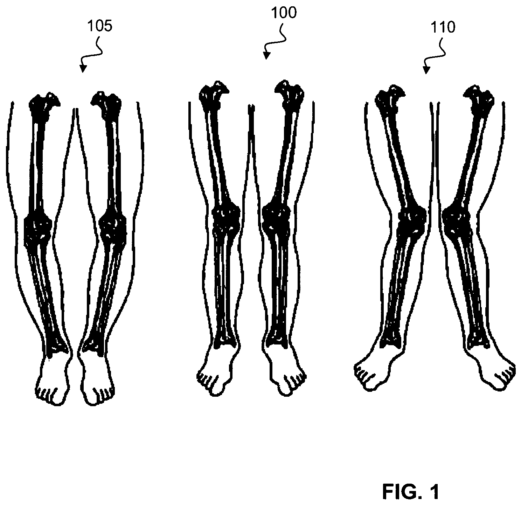

Although osteotomy realignment surgery can be performed on various joints, including the knee, hip, ankle, or shoulder, the most common form of osteotomy realignment surgery is knee realignment surgery. Knee realignment surgery involves cutting and repositioning bones (usually the femur or tibia) to realign the bones around the knee. FIG. 1 is a schematic representation of different circumstances in the knee and associated bones. In particular, circumstance 100 schematically represents a healthy alignment of the bones around the knee, and knee realignment surgery is unnecessary. Circumstance 105 schematically represents a "bow-legged" or varus knee for which knee realignment surgery may be recommended. Circumstance 110 schematically represents a "knock-kneed" or valgus knee for which (a different) knee realignment surgery may be recommended. When knee realignment surgery is performed on patients with circumstances 105, 110, the surgeon seeks to realign the bones at the knee to more closely resemble the healthy alignment of circumstance 100.

Knee and other realignment surgeries are generally planned preoperatively to ensure the procedure's success. Preoperative planning allows orthopedic surgeons to develop a surgical strategy tailored to the individual patient prior to the surgical procedure.

Typically, orthopedic surgeries are planned using 2D X-ray radiographs. For example, knee realignment surgeries are commonly planned using 2D weight-bearing long-leg radiographs (LLR). Such long-leg radiographs are "weight-bearing" in that the radiographic images are acquired while the knee is bearing the weight of the patient, most commonly while the patient is standing. The information provided by such long-leg radiographs may provide different insights than images acquired while the patient is prone or otherwise disposed in a position in which the knee does not bear the weight of the patient. In particular, deformities or misalignments at the knee may be most impactful when the knee and the leg are in a load-bearing position. The surgical procedure can be planned with those impacts in mind.

Nevertheless, using 2D X-ray radiographs to plan orthopedic surgeries presents certain limitations. One is the absence of 3D information, which may impede the identification of underlying anatomical structures or hinder the understanding of bone geometry and integrity. Another is poor soft tissue contrast. The identification of certain pathological conditions in bones and joints is often difficult without high-contrast images of soft tissue.

3D medical images can also be used to plan orthopedic surgeries. Computerized Tomography (CT), Positron Emission Tomography (PET), and Magnetic Resonance Imaging (MRI) are medical imaging techniques that can generate detailed 3D images of anatomical parts. However, these techniques also present certain limitations. Often, a patient must be prone during the acquisition of 3D images. Also, CT and PET scanning require a relatively large exposure to radiation that can potentially be harmful to human cells and increase the incidence of cancer. Another limitation is the price of the equipment and the cost of each scan. 3D medical imaging techniques are generally more expensive than 2D X-ray technology and may not be available in certain contexts.

### SUMMARY

The invention generally relates to the generation of three-dimensional (3D) representations of a patient's anatomy for realignment surgery using long-leg radiographs and, in general, machine-learning systems. The 3D representations can be used for diagnostic evaluation, preoperative planning, intraoperative guidance, and postoperative assessment of realignment surgeries. The 3D representations can be displayed to represent joints, bones, and other anatomical structures. The anatomical structures can be those that are relevant to joint realignment surgery, including, for example, knee, hip, ankle, and shoulder. An Artificial Intelligence (AI) model may be implemented for generating 3D bone models from 2D medical images, e.g., using deep learning or other approaches, as described in EP4239581A1. The model for generating 3D representations of anatomical structures for realignment surgery can be used in conjunction with other AI models that use deep learning and other approaches to perform the methods and workflows described herein.

In a first aspect, a computer-implemented method includes receiving a radiograph that represents a portion of a joint, inferring a 3D structure of the represented portion of the joint from the radiograph, registering the inferred 3D structure of the represented portion of the joint with a weight-bearing radiograph of the portion of the joint, and outputting information characterizing the registration of the inferred 3D structure with the weight-bearing radiograph.

In a second aspect, a computer-implemented method includes receiving one or more radiographs that represent at least a portion of a hip joint of a patient, at least a portion of a knee joint of the patient, and at least a portion of an ankle joint of the patient, inferring 3D structures of the represented portions of the hip joint, of the knee joint, and of the ankle joint, registering the inferred 3D structures with a long-leg radiograph that represents at least a portion of the hip joint of the patient, at least a portion of the knee joint of the patient, and at least a portion of the ankle joint of the patient, and outputting information characterizing the registration of the inferred 3D structures with the long-leg radiograph.

Either the first or the second aspect includes one or more of the following features. The radiograph from which the 3D structure of the represented portion of the joint is inferred is the weight-bearing radiograph of the portion of the joint with which the inferred 3D structure is registered. Registering the inferred 3D structure of the represented portion of the joint with the weight-bearing radiograph of the portion of the joint includes transforming the inferred 3D structure into a 3D mesh, transforming the portion of the joint in the weight-bearing radiograph into a 2D mesh, and registering the 3D mesh with the 2D mesh. Receiving the radiograph includes receiving a plurality of radiographs that represent the portion of the joint. Inferring the 3D structure includes inferring the 3D structure of the represented portion of the joint from the plurality of the radiographs. Receiving the radiograph includes receiving a radiograph that represents a plurality of portions of joints. The method further includes cropping the radiograph that represents a plurality of portions of joints into a plurality of regions, each of which represents one of the plurality of portions of the joints. Inferring the 3D structure of the represented portion of the joint from the radiograph includes inferring the 3D structure using a machine-learning model that has been trained to use radiographs to inferring 3D structures of joints using 3D medical images of the joints as the desired output. The method is a method for generating data suitable for preoperative planning of knee realignment surgery. Receiving the radiograph includes receiving a first long-leg radiograph that represents at least a portion of the hip joint and ankle joint. Receiving the radiograph includes receiving a radiograph of an anteroposterior (AP) view of the knee and receiving a radiograph of a lateral (LAT) view of the knee. Inferring the 3D structure includes inferring the 3D structure of the knee from at least the radiograph of an anteroposterior (AP) view of the knee and the radiograph of a lateral (LAT) view of the knee and registering the inferred 3D structure of the knee with a weight-bearing radiograph of the leg. The received radiograph represents the portion of the joint in a non-weight bearing pose.

Inferring the 3D structure includes inferring a plurality of 3D structures of a plurality of represented portion of joints. Outputting the information includes outputting information characterizing the registration of the plurality of 3D structures with the weight-bearing radiograph. Outputting the information characterizing the registration of the 3D structure includes outputting an image representing the 3D structure in registration with the weight-bearing radiograph. The long-leg radiograph is a weight-bearing long-leg radiograph. Registering the inferred 3D structures with the with a long-leg radiograph includes transforming the inferred 3D structures into 3D meshes, transforming the portions of the hip joint, of the knee joint, and of the ankle joint in the weight-bearing radiograph into a plurality of 2D meshes, and registering the 3D meshes with corresponding of the 2D meshes.

One or more storage devices can store instructions that, when executed by the one or more processors, cause the one or more processors to perform any of the aspects described above.

The details of one or more implementations are set forth in the accompanying drawings and the description below. Other features, objects, and advantages will be apparent from the description and drawings and from the claims.

### DESCRIPTION OF DRAWINGS

FIG. 1 is a schematic representation of different circumstances in the knee and associated bones.
FIG. 2 is a flowchart of a process for generating data that is useful for preoperative planning of orthopedic surgery.
FIG. 3 schematically represents division of a single long-leg radiograph into different regions and the inference of the 3D structures of joints in those regions.
FIGS. 4 and 5 schematically represent example workflows of inference modules for generating 2D and 3D meshes from 2D medical images.
FIG. 6 schematically represents an example workflow of a registration module for aligning and spatially correlate 3D meshes with corresponding 2D representations.

Like reference symbols in the various drawings indicate like elements.

### DETAILED DESCRIPTION

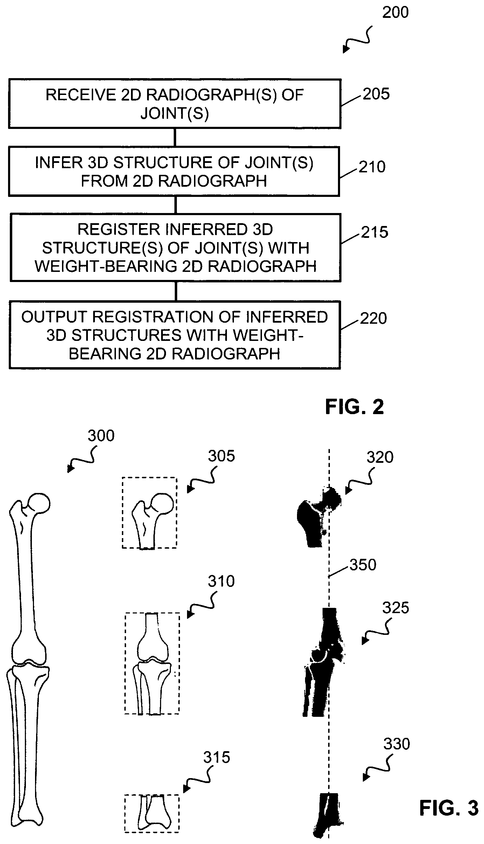

FIG. 2 is a flowchart of a process 200 for generating data that is useful for preoperative planning of orthopedic surgery. The activities of process 200 can be performed by one or more data processing devices that perform data processing activities, e.g., in accordance with the logic of a set of machine-readable instructions, instructions embodied in hardware, or a combination of these and/or other instructions. Process 200 can be performed in isolation or in conjunction with other activities.

At 205, the system performing process 200 receives one or more radiographs of at least one joint. A single joint can be represented in one or more radiographs. Alternatively, multiple joints can be represented in a single radiograph, or multiple joints can be separately represented in different radiographs. Combinations of single radiographs with multiple joints or separate radiographs with individual joints are also possible.

At least one of the radiographs is a weight-bearing radiograph in that the radiographic image is acquired while a represented joint is bearing a physiologically-relevant weight. For example, in the context of surgical realignment of the shoulder, 2D radiograph(s) may be captured while the shoulder is in a position that causes pain or that reflects daily activities of the patient. As another example, in the context of knee realignment surgery, the 2D radiograph(s) may be one or more images of the hip, knee, and ankle that are acquired while the patient is standing.

In some implementations, at least one of the radiographs represents a portion of a joint in a non-weight bearing pose. For example, such a radiographs could have been acquired while the patient was prone.

In some implementations, at least one of the radiographs includes multiple joints. For example, in the context of knee realignment surgery, long-leg radiographs may include the hip, knee, and ankle of a patient.

At 210, the system performing process 200 infers the 3D structure of at least a portion of at least one joint from one or more of the received radiographs. The inference can be performed by an AI model that has been trained to infer 3D structures of joints from radiographs of those joints. For example, a generative AI model can be trained to generate such 3D structures from 2D projections. The training pairs can use radiographs of different joints as inputs and 3D structures of at least a portion of those joints as corresponding outputs. The 3D images can be gathered using any of the 3D medical imaging techniques described above. In some implementations, the inference can be performed using the systems and techniques described in EP 4239581 A1 and EP 4300427 A1, the contents of both of which are incorporated herein by reference.

In some implementations, the inference encompasses individual joints or portions of individual joints without encompassing all of the physiological structures that are represented in a 2D radiograph. The physiological structures not included in the inference may include, as the case may be, other joints that are represented in the 2D radiograph, as well as bones and other structures. In some implementations, a single 2D radiograph may serve as the basis for inferring the 3D structures of multiple joints or portions of joints individually, e.g., one-by-one in series or at the same time, in parallel. To encompass individual joints or portions of individual joints without encompassing all of the physiological structures that are represented in a 2D radiograph, the 2D radiograph can be divided or cropped into different regions that represent an individual joint or portions thereof without encompassing all of the represented physiological structures. The 3D structure of the represented joint/joint portion can then be inferred from the region that represents the joint/joint portion. In some implementations, the 2D radiograph is divided or cropped automatically by the system without active human participation. For example, the system can use image recognition and processing techniques to identify the joint/joint portion and surrounding area. In other implementations, the 2D radiograph is divided or cropped manually by a human user.

In the context of knee realignment surgery, 3D structures of portions or all of each of the hip, knee, and ankle may be individually inferred from one or more radiographs in which they are represented. In some implementations, the inference of a single one of hip, knee, or ankle is based on multiple radiographs. In other implementations, the inference of all three is based on a single long-leg radiograph in which all are represented or combinations such a single long-leg radiograph with other radiographs. In either case, the single long-leg radiograph may be divided or cropped into:
a first region in which the hip is represented but that excludes the knee and ankle,
a second region in which the knee is represented but that excludes the hip and ankle, and
a third region in which the ankle is represented but that excludes the hip and knee. Such regions need not overlay each other or even abut one another in the source radiograph.

For example, FIG. 3 schematically represents the division of a single long-leg radiograph 300 into different regions and the inference of the 3D structures of joints in those regions. Long-leg radiograph 300 can be, e.g., a weight-bearing long-leg radiograph that represents portions of the hip, knee, and ankle. In the illustrated implementation, long-leg radiograph 300 is divided or cropped into a first region 305 that represents a portion of the hip, a second region 310 that represents the entire knee, and a third region 315 that represents a portion of the ankle. Regions 305, 310, and 315 neither overlay nor abut one another in radiograph 300, i.e., there are swathes of radiograph 300 that do not appear in any of regions 305, 310, 315.

Each of the regions 305, 315 is used to infer the corresponding 3D structure of at least a portion of the respective joint that is represented therein-alone or in combination with other radiographs. In the illustrated implementation, first region 305 is used to infer the 3D structure 320 of a portion of the hip (namely, the femoral head, neck, and greater trochanter) and an adjacent portion of the femur. Second region 310 is used to infer the 3D structure 325 of the knee joint and adjacent portions of the femur, fibula, and tibia. Third region 315 is used to infer the 3D structure 330 of a portion of the ankle (namely, the lateral and medial malleolus) and adjacent portions of the fibula and tibia.

Returning to FIG. 2, the system performing process 200 registers the inferred 3D structure(s) of the joint(s) or joint portion(s) with a weight-bearing, long-leg radiograph. The weight-bearing, long-leg radiograph could have been amongst the radiographs used to infer the corresponding 3D structures, but this is not necessarily the case. The registration matches the positions and orientations of the inferred 3D joint(s) or joint portion(s) to the positions and orientations of the corresponding joint(s) or joint portion(s) in the weight-bearing radiograph. Returning briefly to FIG. 3, a line 350 schematically represents registration of the inferred 3D structure(s) of the joint(s) or joint portion(s) with the corresponding structures of the weight-bearing radiograph. Although line 350 is represented in FIG. 3 in the two-dimensional plane of the page, the inferred 3D structure(s) can be registered in three dimensions with the weight-bearing radiograph. Thus, the positions and orientations of the inferred 3D joint(s) or joint portion(s) are matched to the positions and orientations of the corresponding joint(s) or joint portion(s) in the coronal plane, in the axial plane, and in the sagittal plane.

In some implementations, the weight-bearing radiograph with which the inferred 3D structure(s) is registered is the same 2D radiograph from which the 3D structure(s) of the joint or joint portions were inferred at 210. In other implementations, the weight-bearing radiograph with which the inferred 3D structure(s) is registered was one of multiple radiographs from which the 3D structure(s) of the joint or joint portions were inferred at 210. In still other implementations, the 2D radiograph(s) from which the 3D structure(s) of the joint or joint portions were inferred at 210 differ from the weight-bearing radiograph with which the inferred 3D structure(s) are registered.

The registration process can be performed in a number of different ways.

For example, in some implementations, the inferred 3D structures are projected onto 2D from several different poses, as if they were being radiographically imaged under the same or similar conditions as the conditions used when forming the weight-bearing radiograph. The calculated projections of the inferred 3D structures can then be registered with the real-world projections in the weight-bearing radiograph.

As another example, the weight-bearing radiograph can itself be used to infer different 3D structure(s) of the joint or joint portions. These new, different 3D structure(s) can then be registered with the 3D structures that were inferred at 210. Such a registration process is particularly useful when the inference at 210 was performed using 2D radiograph(s) that differ from the weight-bearing radiograph with which the inferred 3D structure(s) are being registered or when the inference at 210 was performed using multiple radiographs, of which the weight-bearing radiograph was only one.

As another example, the registration is performed on mesh models generated from the inferred 3D structures and radiographs. The meshes can subdivide the continuous geometric space in the inferred 3D structures and radiographs into discrete geometric and topological cells. In some implementations, the registration process is performed using a metaheuristic optimization process that registers 2D meshes formed from the radiographs with 3D meshes formed from the inferred 3D structures. A more detailed example of such a registration is described below.

At 220, the system performing process 200 outputs the registration of inferred 3D structure(s) of the joint or joint portions with the weight-bearing radiograph(s). The output can include a graphical representation of the inferred 3D structure(s) of the joint or joint portions in registration with the weight-bearing radiograph and/or data suitable for forming such a graphical representation.

Such graphical representations are particularly useful for preoperative planning of orthopedic surgery, as well as post-operative assessment of orthopedic surgery, diagnoses, and even intraoperatively. In particular, a surgeon or other user is able to visualize the most relevant 3D structure(s)-namely, joints or joint portions- in weight-bearing positions in which deformities or misalignments may be most impactful. In some cases, those 3D structure(s) can be inferred from multiple images and/or higher quality images than the weight-bearing radiograph with which they are subsequently registered. In some cases, the images from which the 3D structure(s) are acquired are in positions that are not weight-bearing but nevertheless provide relevant insight into the joints or joint portions that are inferred therefrom. As a result of these and other advantages, the quality of the planning and post-surgical outcomes can be improved.

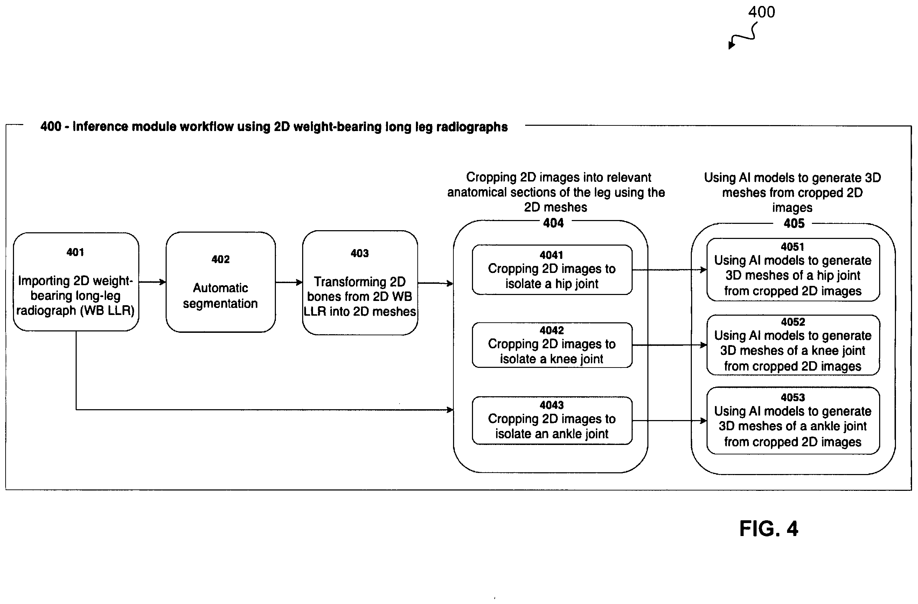

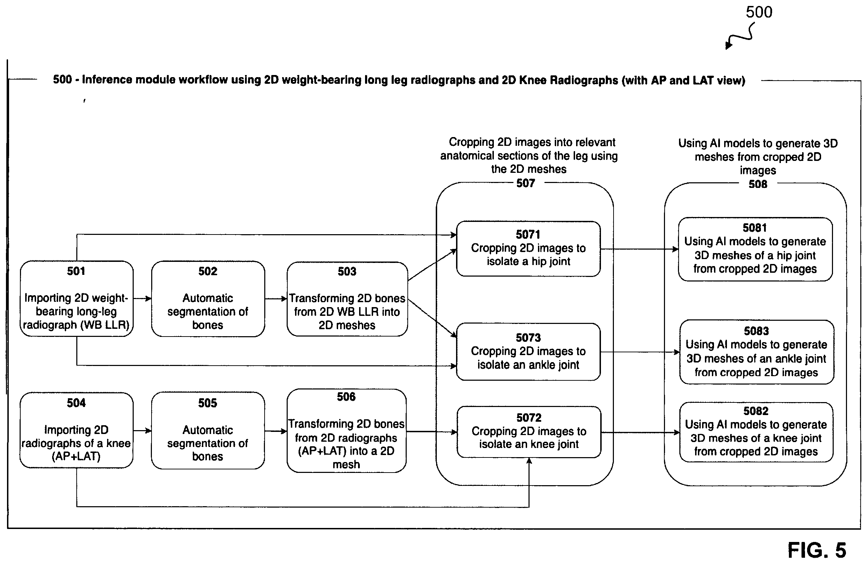

FIGS. 4 and 5 schematically represent example workflows 400, 500 of inference modules for generating 2D and 3D meshes from 2D medical images. Either of workflows 400, 500 can be used to perform the inference at 210 in process 200. Workflows 400, 500 are both described in the context of knee realignment surgery. However, the techniques described therein can be used in other osteotomy realignment surgeries and in orthopedic surgery in general.

Workflows 400, 500 differ in that the inference module performing workflow 400 uses 2D Weight-Bearing Long-Leg Radiographs to perform the inference. In contrast, the inference module performing workflow 500 uses a 2D radiograph of a knee joint in an anteroposterior view and a 2D radiograph of a knee joint in a lateral view in conjunction with 2D Weight-Bearing Long-Leg Radiographs from the same patient to perform the inference. A user or operator may selectively determine which medical images are to be imported into the inference module. Regardless of the radiographs used, the inference modules that implement workflows 400, 500 generate 3D meshes of relevant anatomical parts.

Referring first to workflow 400 of FIG. 4, at 401, the system performing workflow 400 imports at least one 2D weight-bearing (WB) Long-Leg Radiograph (LLR) of a patient (401). The imported 2D WB LLR(s) can include an anteroposterior (AP) view. In some implementations, an additional 2D WB LLR of a lateral (LAT) view can also be imported. 2D WB LLRs represent anatomical structures of the lower limbs of a patient in two dimensions. Anatomical structures of lower limbs include at least a hip joint, femur, knee joint, tibia, and ankle joint.

The 2D WB LLR(s) can be in any of a variety of different image formats. For example, the images can be Portable Network Graphics (*.png) images, joint photographic group (*.jpg) images, tagged image file format (*.tiff) etc. images, DICOM images, or the like.

In the illustrated implementation, at 402, the system performing workflow 400 automatically (i.e., without the involvement of a human) segments the imported 2D WB LLR(s) to identify bones that are represented in those 2D WB LLR(s). The segmentation can identify regions of the imported 2D WB LLR(s) that represent bones based on the contrast between the bones and soft tissue, based on the expected anatomical geometry of the bones themselves, and/or using a machine learning model that has been trained to perform such segmentations. In the context of knee realignment surgery, the system can automatically identify regions of the 2D WB LLR(s) that represent the femur and tibia. In other implementations, the segmentation is manual or human-guided.

At 403, the system performing workflow 400 transforms at least a portion of the 2D representations of the bones-including but not necessarily limited to the portions of the bones that form joints- from the 2D WB LLR(s) into 2D meshes. The meshes can be structured representations of the bones and include interconnected elements that define the bone's shape and structure. In 2D meshes, the interconnected elements generally include vertices, edges, and surfaces representing an object, e.g., representing the bones.

At 404, the system performing workflow 400 crops the 2D WB LLR(s) to select regions of the 2D WB LLR(s) that represent relevant sections of the leg. In the illustrated implementation, the system crops the 2D WB LLR(s) using the 2D meshes to isolate the hip, knee, and ankle joints in the selected regions. In particular, the hip, knee, and ankle joints are first identified in the 2D meshes using the shape and structure information that is embodied in the meshes. The spatial information of the 2D meshes can be used to identify locations of the hip, knee, and ankle joints in the 2D WB LLR(s), and crop them accordingly.

The relevant sections are those that provide information relevant to the realignment surgery. In the context of knee realignment surgery, the relevant sections of the leg include portions of the hip joint, the knee joint, and the ankle joint. These sections can be used for diagnostic purposes, for preoperative planning, and serve as references for intraoperative and postoperative assessments. In some implementations, the relevant sections of the hip joint include the femoral head, the femoral neck, and a proximal portion of the femoral shaft. In some implementations, the relevant sections of the hip joint also include the proximal section of a femur. In some implementations, the relevant sections of the knee joint include the articular zone, the distal end of the femur, and the proximal end of the tibia, including their corresponding articular surfaces. In some implementations, the relevant sections of the ankle joint include the distal section of the tibia. In some implementations, the relevant sections of the ankle joint also include the tibial plafond, the medial malleolus, and the distal shaft of the tibia. In the illustrated implementation in the context of knee realignment surgery, the system crops the 2D WB LLR(s) to select regions of the 2D WB LLR(s) that represent the hip joint at 4041, the knee joint at 4042, and of the ankle joint at 4043.

At 405, the system performing workflow 400 transforms the regions cropped from the 2D WB LLR(s) into 3D meshes using AI models. 3D meshes include volumetric elements that represent 3D objects (e.g., bone) and include vertices, edges, surfaces, and volumes. The transformation can be a two-step process in which the regions cropped from the 2D WB LLR(s) are first transformed into 3D images and those images into 3D meshes. A method for transforming 2D images into 3D images is disclosed in European Application No. 22398005.3, filed on March 4, 2022, the contents of which are incorporated herein by reference.

Since the system transforms the cropped regions of the 2D WB LLR(s) into 3D meshes, the system generates 3D meshes of the relevant anatomical parts. The relevant sections are those that provide information relevant to the realignment surgery. In the illustrated implementation in the context of knee realignment surgery, the system generates 3D meshes of the hip joint at 4051, of the knee joint at 4052, and of the ankle joint at 4053.

The 3D meshes can be output and used in downstream data processing activities. For example, in the context of process 200 (FIG. 2), the 3D meshes can be registered with a weight-bearing radiograph at 215.

Referring to workflow 500 of FIG. 5, at 501, the system performing workflow 500 imports at least one 2D WB LLR of a patient and, at 504, imports radiographs of the knee joint from the same patient in AP and LAT views. The 2D WB LLR imported at 501 can be an anteroposterior (AP) view 2D WB LLR. In some implementations, an additional, lateral (LAT) view 2D WB LLR. The radiographs of a knee joint in AP and LAT views imported at 504 are X-ray projections of a knee joint along the frontal plane (AP) and along the sagittal plane (LAT). The AP and LAT views 2D imported at 504 are of the same patient as the 2D WB LLR imported at 501. The imported images can be in any of a variety of different image formats.

In the illustrated implementation, at 502, the system performing workflow 500 automatically (i.e., without the involvement of a human) segments the imported 2D WB LLR(s) to identify bones that are represented in those 2D WB LLR(s). The segmentation at 502 can be performed in the same manner as the segmentation at 402 in process 400 (FIG. 4).

At 503, the system performing workflow 500 transforms at least a portion of the 2D representations of the bones-including but not necessarily limited to the portions of the bones that form joints- from the 2D WB LLR(s) into 2D meshes. The transformation at 503 can be performed in the same manner as the transformation at 403 in process 400 (FIG. 4).

At 505, the system performing workflow 500 automatically (i.e., without the involvement of a human) segments the AP and LAT view radiographs of the knee joint. The segmentation at 505 can be performed in the same manner as the segmentation at 403 in process 400 (FIG. 4).

At 506, the system performing workflow 500 transforms at least a portion of the 2D representations of the bones-including but not necessarily limited to the portions of the bones that form joints- from the AP and LAT view radiographs into 2D meshes. The transformation at 503 can be performed in the same manner as the transformation at 403 in process 400 (FIG. 4).

At 507, the system performing workflow 500 crops the 2D WB LLR(s) and the AP and LAT view radiographs into relevant sections of the leg using the 2D meshes to isolate the hip, knee, and ankle joints in the selected regions. In the illustrated implementation, the system crops the 2D WB LLR(s) using the 2D meshes into which the 2D representations of the bones from the 2D WB LLR(s) were transformed to isolate, in the selected regions, the hip joint at 5071 and ankle joint at 5073. Also, the system crops the AP and LAT view radiographs using the 2D meshes into which the 2D representations of the bones from the AP and LAT view radiographs were transformed to isolate the knee joint at 5072 in the selected regions. The hip, knee, and ankle joints are first identified in the 2D meshes using the shape and structure information that is embodied in the meshes. The spatial information of the 2D meshes can be used to identify locations of the hip, knee, and ankle joints in the AP and LAT view radiographs and 2D WB LLR(s), and crop them accordingly.

At 508, the system performing workflow 500 transforms the regions cropped from the AP and LAT view radiographs and the 2D WB LLR(s) into 3D meshes using AI models. The transformation at 208 can be performed in the same manner as the transformation at 403 in process 400 (FIG. 4).

Since the system performing workflow 500 transforms the cropped regions of the 2D WB LLR(s) into 3D meshes, the system generates meshes of the relevant anatomical parts. In the illustrated implementation in the context of knee realignment surgery, the system generates 3D meshes of the hip joint at 5081, of the knee joint at 5082, and of the ankle joint at 5083. The meshes can be generated at 508 in the same manner as the meshes are generated at 405 in process 400 (FIG. 4).

The 3D meshes can be output and used in downstream data processing activities. For example, in the context of process 200 (FIG. 2), the 3D meshes can be registered with a weight-bearing radiograph at 215.

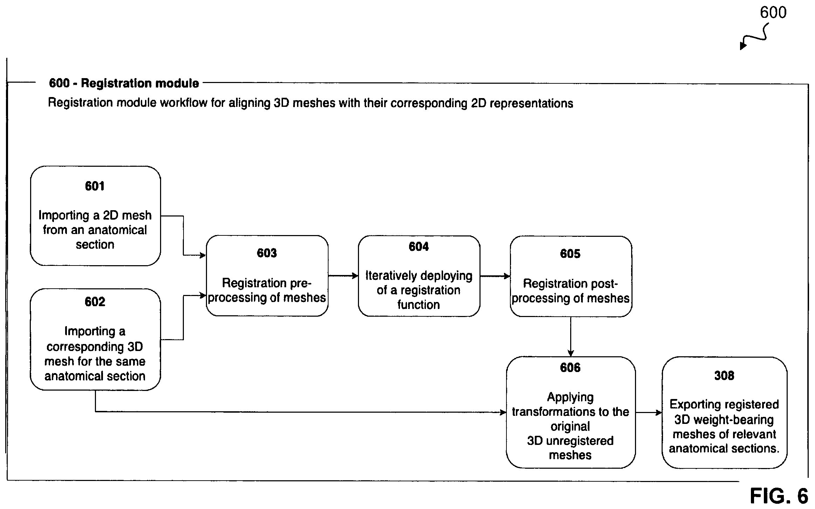

FIG. 6 schematically represents an example workflow 600 of a registration module for aligning and spatially correlate 3D meshes with corresponding 2D representations. Workflow 600 can be used to perform the registration at 215 in process 200 (FIG. 2). The 3D meshes used in workflow 600 can be the 3D meshes output from either of workflows 400, 500 (FIGS. 4, 5). For example, the 3D meshes generated at 405 or 508 in workflows 400, 500 can be registered with the 2D meshes generated at 403 or 503, 506, and then with the 2D WB LLR(s) and AP and LAT view radiographs from which these 2D meshes were generated.

Workflow 600 is described in the context of knee realignment surgery. However, the techniques described therein can be used in other osteotomy realignment surgeries and in orthopedic surgery in general. The registration in process 600 may be metaheuristic optimization. The 3D meshes and their corresponding 2D representations generally originate from the same patient and the same anatomical region, although this is not necessarily the case.

At 601, the system performing workflow 600 imports at least one 2D mesh from an anatomical section. The 2D mesh may be a 2D mesh generated by an inference module, e.g., the 2D mesh may be generated at 403, 503, 506 in workflows 400, 500.

At 602, the system performing workflow 600 also imports at least one 3D mesh. Each 3D mesh represents the same anatomical section as a corresponding 2D mesh that was imported at 601. The imported 3D mesh(es) may have been generated by an inference module, e.g., the 3D mesh(es) may have been generated at 405 or 508 in workflows 400, 500.

In some implementations, a 2D mesh of an anatomical region of a patient and the corresponding 3D mesh of the same anatomical region of the same patient may be imported at 601, 602, respectively.

At 603, the system performing workflow 600 pre-processes the 2D and 3D meshes imported at 601, 602. In some implementations, the pre-processing includes cropping to isolate relevant parts of the 2D mesh(es) and corresponding relevant parts of the 3D mesh(es). For example, if the system aims at registering the hip joint, the region or volumes that represent the hip joint will be cropped from a 2D mesh and a corresponding 3D mesh. In some implementations, pre-processing can also include an initial registration estimation and/or a data-type conversion from meshes to binary matrices. The pre-processing yields pre-processed 2D meshes and pre-processed 3D meshes.

At 604, the system performing workflow 600 iteratively employs a registration function to align the pre-processed 2D meshes with the pre-processed 3D meshes. In some implementations, the registration function applies a spatial transformation to one or both of corresponding pre-processed 2D and 3D meshes and evaluates the quality of the registration. The application of a spatial transformation generally includes translation and/or rotation of a 3D pre-processed mesh to align features represented therein with corresponding features represented in a 2D pre-processed mesh.

The iterative employment of the registration function may rely upon assessments of proposed registrations and iterative improvements to the proposed registrations until an acceptable registration is reached. For example, a metaheuristic optimization algorithm or other methods may be used to iteratively adjust the parameters of a spatial transformation to improve the alignment of the pre-processed meshes. The registration function may be repeated until a threshold level of registration is achieved.

In some implementations, the metaheuristic optimization algorithm uses a swarm of particles, each representing a potential solution, which updates its position based on its own performance and a best-known solution, thereby converging toward a better alignment. Such a metaheuristic optimization algorithm may be used to find an acceptable position and orientation for the alignment of an object in a 3D pre-processed mesh with a corresponding object in a 2D pre-processed mesh.

At 605, the system performing workflow 600 post-processes the aligned 3D meshes to determine the operations that are needed to map the 3D meshes from the original positioning to the positioning in which they are registered with their corresponding 2D meshes. The processing is thus after (or post) the registration of the meshes. In some implementations, the registration post-processing determines a transformation matrix that transforms the original 3D meshes into 3D meshes that are aligned with their corresponding 2D meshes. The transformation matrix may encode operations such as translation and rotation. The transformation matrix may apply linear transformations to manipulate the 3D meshes in space. The manipulations of the spatial data may include positional offsets, orientation, and scaling factors that are encoded in the transformation matrix. The transformation matrix can thus represent the operations required to map a 3D mesh from its original configuration to an intended alignment or spatial positioning in which it is registered with a corresponding 2D mesh.

At 606, the system performing workflow 600 applies the operations determined by the post-processing at 605 to the entirety of the 3D unregistered 3D mesh(es) imported at 602. Since the 3D meshes imported at 602 were preprocessed at 603, the operations determined by the post-processing at 605 were determined using only a portion of the unregistered 3D mesh(es) imported at 602. However, those same operations are now applied to the entirety of the 3D unregistered 3D mesh(es).

The application of the operations determined by the post-processing at 605 repositions the unregistered 3D mesh(es) imported at 602 to their weight-bearing position. The repositioning thus relies on registration of portions of those original, unregistered 3D mesh(es) with their corresponding unregistered 2D meshes to reflect the weight-bearing information when the entirety of the 3D unregistered 3D mesh(es) are repositioned.

At 607, the system performing workflow 600 exports the 3D weight-bearing 3D meshes to which the registration operations have been applied.

In instances where 3D meshes of a hip joint (generated, e.g., at 4051 or 5081 in processes 400, 500) are imported at 602, workflow 600 will export a registered 3D weight-bearing mesh of a hip joint at 607.

In instances where 3D meshes of a knee joint (generated, e.g., at 4052 or 5082 in processes 400, 500) are imported at 602, workflow 600 will export a registered 3D weight-bearing mesh of a knee joint at 607.

In instances where 3D meshes of an ankle joint (generated, e.g., at 4053 or 5083 in processes 400, 500) are imported at 602, workflow 600 will export a registered 3D weight-bearing mesh of an ankle joint at 607.

As a result, the 3D meshes of the relevant physiological features (here, hip joint, knee joint, and ankle joint) will be registered with their positions in a weight-bearing image. The resulting aligned registered 3D weight-bearing mesh can be used in several contexts that are relevant to orthopedic surgery, including diagnosis, preoperative planning, intraoperative guidance, and postoperative assessments.

Implementations of the subject matter and the operations described in this specification can be implemented in digital electronic circuitry, or in computer software, firmware, or hardware, including the structures disclosed in this specification and their structural equivalents, or in combinations of one or more of them. Implementations described herein can be implemented as one or more computer programs, i.e., one or more modules of computer program instructions, encoded on computer storage medium for execution by, or to control the operation of, data processing apparatus. Alternatively or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

The operations described herein can be implemented as operations performed by a data processing apparatus on data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes, workflows, and logic flows described herein can be performed by one or more programmable processors executing one or more computer programs to perform actions by operating on input data and generating output. The processes, workflows, and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, implementations can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

A number of implementations have been described. Nevertheless, it will be understood that various modifications may be made. Accordingly, other implementations are within the scope of the following claims.

## Claims

1. A computer-implemented method comprising:
receiving a radiograph that represents a portion of a joint;
inferring a 3D structure of the represented portion of the joint from the radiograph;
registering the inferred 3D structure of the represented portion of the joint with a weight-bearing radiograph of the portion of the joint; and
outputting information characterizing the registration of the inferred 3D structure with the weight-bearing radiograph.

2. The method of claim 1, wherein the radiograph from which the 3D structure of the represented portion of the joint is inferred is the weight-bearing radiograph of the portion of the joint with which the inferred 3D structure is registered.

3. The method of any preceding claim, wherein registering the inferred 3D structure of the represented portion of the joint with the weight-bearing radiograph of the portion of the joint comprises:
transforming the inferred 3D structure into a 3D mesh;
transforming the portion of the joint in the weight-bearing radiograph into a 2D mesh; and
registering the 3D mesh with the 2D mesh.

4. The method of any preceding claim, wherein:
receiving the radiograph comprises receiving a plurality of radiographs that represent the portion of the joint; and
inferring the 3D structure comprises inferring the 3D structure of the represented portion of the joint from the plurality of the radiographs.

5. The method of any preceding claim, wherein receiving the radiograph comprises receiving a radiograph that represents a plurality of portions of joints, and
optionally wherein the method further comprises cropping the radiograph that represents a plurality of portions of joints into a plurality of regions, each of which represents one of the plurality of portions of the joints.

6. The method of any preceding claim, wherein inferring the 3D structure of the represented portion of the joint from the radiograph comprises inferring the 3D structure using a machine-learning model that has been trained to use radiographs to inferring 3D structures of joints using 3D medical images of the joints as the desired output.

7. The method of any preceding claim, wherein the method is a method for generating data suitable for preoperative planning of knee realignment surgery.

8. The method of any preceding claim, wherein receiving the radiograph comprises receiving a first long-leg radiograph that represents at least a portion of the hip joint and ankle joint.

9. The method of any preceding claim, wherein receiving the radiograph comprises receiving a radiograph of an anteroposterior (AP) view of the knee and receiving a radiograph of a lateral (LAT) view of the knee, and
optionally wherein
inferring the 3D structure comprises inferring the 3D structure of the knee from at least the radiograph of an anteroposterior (AP) view of the knee and the radiograph of a lateral (LAT) view of the knee and
registering the inferred 3D structure of the knee with a weight-bearing radiograph of the leg.

10. The method of any preceding claim, wherein:
inferring the 3D structure comprises inferring a plurality of 3D structures of a plurality of represented portion of joints; and
outputting the information comprises outputting information characterizing the registration of the plurality of 3D structures with the weight-bearing radiograph.

11. The method of any preceding claim, wherein outputting the information characterizing the registration of the 3D structure comprises outputting an image representing the 3D structure in registration with the weight-bearing radiograph.

12. The method of any preceding claim, wherein the received radiograph represents the portion of the joint in a non-weight bearing pose.

13. A computer-implemented method comprising:
receiving one or more radiographs that represent at least a portion of a hip joint of a patient, at least a portion of a knee joint of the patient, and at least a portion of an ankle joint of the patient;
inferring 3D structures of the represented portions of the hip joint, of the knee joint, and of the ankle joint;
registering the inferred 3D structures with a long-leg radiograph that represents at least a portion of the hip joint of the patient, at least a portion of the knee joint of the patient, and at least a portion of the ankle joint of the patient; and
outputting information characterizing the registration of the inferred 3D structures with the long-leg radiograph.

14. The computer-implemented method of claim 13, wherein the long-leg radiograph is a weight-bearing long-leg radiograph.

15. The computer-implemented method of claim 13 or 14, wherein registering the inferred 3D structures with a long-leg radiograph comprises:
transforming the inferred 3D structures into 3D meshes;
transforming the portions of the hip joint, of the knee joint, and of the ankle joint in the weight-bearing radiograph into a plurality of 2D meshes; and
registering the 3D meshes with corresponding of the 2D meshes.

16. One or more storage devices, the one or more storage devices storing instructions that, when executed by the one or more processors, cause the one or more processors to perform the operations of any of claims 1 to 15.
